# EUROPEAN PATENT APPLICATION

(11) **EP 4 786 591 A1**
(43) Date of publication of application: **05.08.2026**
(21) Application number: 24873053.3
(22) Date of filing: 27.09.2024
(51) Int. Cl.: C12N 15/11, C12N 15/67, C07K 14/47, A61K 48/00, A61P 35/00, A61K 38/00

(54) **NUCLEIC ACID MOLECULE COMPRISING MODIFIED POLYADENYLATION SEQUENCE AND P53 PROTEIN CODING SEQUENCE**

(30) Priority: 27.09.2023 KR 20230130730
(71) Applicant: Hanmi Pharm. Co., Ltd., Hwaseong-si, Gyeonggi-do 18536 (KR)
(72) Inventor: SHIN, Seung Hyun, Hwaseong-si, Gyeonggi-do 18469 (KR); LIM, Chang Gyu, Hwaseong-si, Gyeonggi-do 18469 (KR); JANG, Doo Seo, Hwaseong-si, Gyeonggi-do 18469 (KR); HAN, Young Jin, Hwaseong-si, Gyeonggi-do 18469 (KR); HEO, Yong Ho, Hwaseong-si, Gyeonggi-do 18469 (KR)
(74) Representative: Santarelli
(86) International application number: PCT/KR2024/014756
(87) International publication number: WO 2025/071346

(57) **Abstract**

Provided are an mRNA molecule including a p53 protein coding sequence and a modified polyadenylation sequence, a composition for delivering the mRNA or a product expressed thereby to a subject, a method of delivering the mRNA or a product expressed thereby to a host cell, and a DNA molecule encoding the mRNA molecule.

## Description

### Technical Field

The present disclosure relates to a nucleic acid molecule including a modified polyadenylation sequence and a p53 coding sequence, a nucleic acid molecule encoding the same, and uses thereof.

### Background Art

RNA, such as *in vitro* transcribed RNA (IVT-RNA), has been proposed as an alternative to the therapeutic use of DNA. However, the injection of RNA for clinical applications may be significantly limited by the short half-life of RNA.

Additionally, IVT vectors may be used as templates for in vitro transcription. Such an IVT vector may include a 5' RNA polymerase promoter, a gene of interest flanked by a 5' UTR, a 3' UTR, or both 5' UTR and 3' UTR, and a 3' polyadenylation cassette including A nucleotides.

The 3' poly(A) of RNA is important for the nuclear export, RNA stability, and translation efficiency of eukaryotic mRNA. It is known that the 3' poly(A) sequence shortens over time, and when it becomes sufficiently short, the RNA is degraded by enzymes.

Therefore, despite the aforementioned prior art, there is a need for a nucleic acid molecule including a modified polyadenylation sequence, a nucleic acid molecule encoding the same, and uses thereof.

### Disclosure

### Technical Problem

An aspect provides an mRNA molecule including a p53 protein coding sequence and a modified polyadenylation sequence.

Another aspect provides a DNA molecule encoding the mRNA molecule.

Another aspect provides a method of propagating the DNA molecule.

Another aspect provides a method of obtaining an RNA.

Another aspect provides a method of obtaining a p53 protein.

Another aspect provides a composition or kit for delivering an mRNA or a product expressed thereby to a subject, including the mRNA molecule.

Another aspect provides a method of delivering the mRNA molecule to a subject.

### Technical Solution

A first aspect provides an mRNA molecule including a p53 protein coding sequence and a modified polyadenylation sequence, wherein the modified polyadenylation sequence includes: (a) a first sequence including one or more microRNA binding sequences; (b) a second sequence of 20 or fewer consecutive A nucleotides, located 5' of the first sequence; and (c) a third sequence of 80 or more consecutive A nucleotides, located 3' of the first sequence.

p53 is a protein encoded by the TP53 gene in humans. It is known that p53 has 15 or more isoforms, and p53 as used herein is interpreted to include these isoforms. p53 is known to prevent genomic mutations by binding to DNA and regulating gene expression. The p53 protein may inhibit tumor formation. The p53 may be a recombinant one, including a fusion protein. The nucleic acid sequence encoding the p53 protein may be codon-optimized. The codon optimization may vary depending on the host cell into which the nucleic acid molecule is introduced. Furthermore, the nucleic acid sequence encoding the p53 protein may include a degenerate sequence.

As used herein, the terms "polyadenyl sequence," "poly(A)," or "poly(A) tail" refer to a region of mRNA downstream of the 3'UTR, for example, directly downstream (i.e., 3' to) the 3'UTR, including a plurality of consecutive adenosine monophosphates. The poly(A) tail may include 10 to 1000 adenosine monophosphates. For example, the poly(A) tail may include 10, 20, 30, 40, 50, 60, 70, 80, 90, 100, 110, 120, 130, 140, 150, 160, 170, 190, 200, 210, 220, 230, 240, 250, 260, 270, 280, 290, 300, 350, 400, 450, 500, 600, 700, 800, 900, or 1,000 or more A nucleotides. The poly(A) tail may have, for example, 50 to 1000, 60 to 800, 80 to 600, 100 to 500, 50 to 500, 60 to 500, 70 to 500, 80 to 500, 90 to 500, 100 to 500, 50 to 400, 60 to 400, 70 to 400, 80 to 400, 90 to 400, 100 to 400, 50 to 300, 60 to 300, 70 to 300, 80 to 300, 90 to 300, 100 to 300, 50 to 200, 50 to 250, 60 to 200, 70 to 200, 80 to 200, 90 to 200, 100 to 200, 50 to 100, 60 to 100, 70 to 100, 80 to 100, 90 to 100, or 150 to 250 consecutive A nucleotides. In vivo, such as in a cell or a subject, the poly(A) tail may serve to protect mRNA from attack by enzymes, for example, enzymes in the cytoplasm, and to aid in transcription termination, nuclear export of mRNA, and translation.

Polyadenylation is the addition of a poly(A) sequence to primary transcript RNA. The poly(A) sequence is composed of a plurality of adenosine monophosphates. That is, a poly(A) sequence is a stretch of RNA containing only adenine bases. The poly(A) sequence is important for nuclear export, translation, and the stability of mRNA. The poly(A) sequence shortens over time, and when it is sufficiently short, the mRNA can be degraded by enzymes.

In the mRNA molecule, the modified polyadenylation sequence may include one or more disruptions in the consecutive A sequence, to which one or more microRNA binding sequences are linked. The modified polyadenylation sequence may perform the original functions of a polyadenylation sequence with equal or superior efficiency compared to an unmodified polyadenylation sequence. The original functions may include aiding in, for example, enhancing, the nuclear export, translation, and stability of mRNA.

As used herein, the term "microRNA" or "miRNA" is used in the sense known in the art. The microRNA may be a long non-coding RNA of 19-25 nucleotides in length that binds to a nucleic acid molecule to downregulate gene expression. The microRNA may bind to the 3'UTR of an mRNA. The microRNA may include a sequence complementary to a region of a target mRNA to which it binds. The complementary sequence may be a perfectly or near-perfectly complementary sequence. The downregulation of gene expression may include downregulation by decreasing the stability of a nucleic acid molecule or by inhibiting translation.

The term "microRNA binding sequence" may be any nucleotide sequence to which a microRNA binds or associates. The term "binding" may follow traditional Watson-Crick hybridization rules or may reflect any stable association between the microRNA and a target sequence at or adjacent to the microRNA binding sequence. The microRNA binding sequence may be a microRNA target sequence, a microRNA sequence, a microRNA seed sequence, or a combination thereof. The microRNA binding sequence may be a known sequence. The microRNA sequence includes a "seed sequence." The seed sequence may be a sequence in the region of positions 2-8 of a mature microRNA. The seed sequence may have complete Watson-Crick complementarity to the microRNA target sequence. An example of the microRNA sequence may include positions 2-8 or 2-7 of a mature microRNA. The microRNA sequence may include 7 nucleotides (e.g., nucleotides 2 to 8 of a mature microRNA), and the seed-complementary site of the corresponding miRNA target may be flanked by an adenine (A) opposite to position 1 of the microRNA. Additionally, the microRNA sequence may include 6 nucleotides (e.g., nucleotides 2 to 7 of a mature microRNA), and the seed-complementary site of the corresponding miRNA target may be flanked by an adenine (A) opposite to position 1 of the microRNA. The microRNA binding sequence may be naturally occurring or artificially synthesized.

In the first sequence of the modified polyadenylation sequence, when two or more microRNA binding sequences are included, they may be identical to or different from each other. Furthermore, when two or more microRNA binding sequences are included, they may have microRNA target sequences, microRNA recognition sequences, or seed sequences thereof that are identical to or different from each other. For example, two or more microRNA binding sequences may be arranged in the form of ABAB, AABB, or ABBA. The two or more microRNA binding sequences may be directly linked to each other without a spacer. Additionally, the two or more microRNA binding sequences may be linked to each other via a spacer. The first sequence may be composed of one or more microRNA binding sequences. The spacer may be a modified or unmodified nucleotide sequence.

In the mRNA molecule, the microRNA may be tumor-specific. The microRNA may be upregulated or downregulated in tumor cells compared to normal cells. The microRNA binding sequence may inhibit a tumor by binding to a tumor-specific miRNA, a seed sequence thereof, or a miRNA target sequence in vivo.

The microRNA may be selected from the group consisting of: miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-34a, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p. An example of the microRNA may be one that is downregulated in tumor cells. The downregulated miR may be miR-34a. However, the differential expression of miRs in cancer cells may vary depending on the type of cancer.

In the second sequence of the modified polyadenylation sequence, the length of the consecutive A nucleotides may be any length that provides sufficient stability and/or translation to the mRNA obtained upon transcription, given the specific microRNA binding sequences, the number thereof, and the length of the consecutive A nucleotides of the third sequence. For example, the length of the consecutive A nucleotides of the second sequence may be 1 to 20, 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, 19 to 20, 20, 1 to 19, 2 to 19, 3 to 19, 4 to 19, 5 to 19, 6 to 19, 7 to 19, 8 to 19, 9 to 19, 10 to 19, 11 to 19, 12 to 19, 13 to 19, 14 to 19, 15 to 19, 16 to 19, 17 to 19, 18 to 19, 19, 1 to 18, 2 to 18, 3 to 18, 4 to 18, 5 to 18, 6 to 18, 7 to 18, 8 to 18, 9 to 18, 10 to 18, 11 to 18, 12 to 18, 13 to 18, 14 to 18, 15 to 18, 16 to 18, 17 to 18, 18, 1 to 17, 2 to 17, 3 to 17, 4 to 17, 5 to 17, 6 to 17, 7 to 17, 8 to 17, 9 to 17, 10 to 17, 11 to 17, 12 to 17, 13 to 17, 14 to 17, 15 to 17, 16 to 17, 17, 1 to 16, 2 to 16, 3 to 16, 4 to 16, 5 to 16, 6 to 16, 7 to 16, 8 to 16, 9 to 16, 10 to 16, 11 to 16, 12 to 16, 13 to 16, 14 to 16, 15 to 16, 16, 1 to 15, 2 to 15, 3 to 15, 4 to 15, 5 to 15, 6 to 15, 7 to 15, 8 to 15, 9 to 15, 10 to 15, 11 to 15, 12 to 15, 13 to 15, 14 to 15, 15, 1 to 14, 2 to 14, 3 to 14, 4 to 14, 5 to 14, 6 to 14, 7 to 14, 8 to 14, 9 to 14, 10 to 14, 11 to 14, 12 to 14, 13 to 14, 14, 1 to 13, 2 to 13, 3 to 13, 4 to 13, 5 to 13, 6 to 13, 7 to 13, 8 to 13, 9 to 13, 10 to 13, 11 to 13, 12 to 13, 13, 1 to 12, 2 to 12, 3 to 12, 4 to 12, 5 to 12, 6 to 12, 7 to 12, 8 to 12, 9 to 12, 10 to 12, 11 to 12, 12, 1 to 11, 2 to 11, 3 to 11, 4 to 11, 5 to 11, 6 to 11, 7 to 11, 8 to 11, 9 to 11, 10 to 11, 11, 1 to 10, 2 to 10, 3 to 10, 4 to 10, 5 to 10, 6 to 10, 7 to 10, 8 to 10, 9 to 10, 10, 1 to 9, 2 to 9, 3 to 9, 4 to 9, 5 to 9, 6 to 9, 7 to 9, 8 to 9, 9, 1 to 8, 2 to 8, 3 to 8, 4 to 8, 5 to 8, 6 to 8, 7 to 8, 8, 1 to 7, 2 to 7, 3 to 7, 4 to 7, 5 to 7, 6 to 7, 7, 1 to 6, 2 to 6, 3 to 6, 4 to 6, 5 to 6, 6, 1 to 5, 2 to 5, 3 to 5, 4 to 5, 5, 1 to 4, 2 to 4, 3 to 4, 4, 1 to 3, 2 to 3, 3, 1 to 2, 2, or 1 nucleotide.

In the third sequence of the modified polyadenylation sequence, the length of the consecutive A nucleotides may be any length that provides sufficient stability and/or translation to the mRNA obtained upon transcription, given the specific microRNA binding sequences, the number thereof, and the length of the consecutive A nucleotides of the second sequence. For example, the length of the consecutive A nucleotides of the third sequence may be 80 to 150, 80 to 140, 80 to 130, 80 to 120, 80 to 110, 90 to 150, 90 to 140, 90 to 130, 90 to 120, or 90 to 110 nucleotides.

In the mRNA molecule, the length of the modified polyadenylation sequence may vary depending on the number of given microRNA binding sequences. For example, the length of the modified polyadenylation sequence may be a length increased by a length corresponding to the number of microRNA binding sequences introduced, relative to the length of the polyadenylation sequence described in the section for "polyadenylation sequence." For example, when the length of the polyadenylation sequence is 10 to 1,000, 20 to 500, 30 to 500, 50 to 100, 80 to 160, 150 to 250, 100 to 300, 40 to 120, or 120 to 200 nucleotides, and when 1, 2, 3, 4, or 5 microRNA binding sequences are included, the modified polyadenylation sequence may have a sequence length of: 29 to 1,025, 39 to 525, 49 to 525, 69 to 125, 99 to 185, 169 to 275, 119 to 325, 59 to 145, 139 to 225, 48 to 1,050, 58 to 550, 68 to 550, 88 to 150, 118 to 210, 188 to 300, 138 to 350, 78 to 170, 158 to 250, 67 to 1,075, 77 to 575, 87 to 575, 107 to 175, 137 to 235, 207 to 325, 157 to 375, 97 to 195, 177 to 275, 86 to 1,100, 96 to 600, 106 to 600, 126 to 200, 156 to 260, 226 to 350, 176 to 400, 116 to 220, 196 to 300, 105 to 1,125, 115 to 625, 125 to 625, 145 to 225, 175 to 285, 245 to 375, 195 to 425, 135 to 245, or 215 to 325 nucleotides. The lengths of the modified polyadenylation sequence are described by way of example and are not limited to the lengths.

In the mRNA molecule, the one or more microRNA binding sequences may be located in a region of positions 21 to 39, 21 to 45, 21 to 70, 21 to 95, 21 to 120, 21 to 145, 11 to 39, 11 to 45, 11 to 70, 11 to 95, 11 to 120, or 11 to 145 of the modified polyadenylation sequence.

Additionally, the mRNA molecule may include an expression control sequence. The expression control sequence may be a sequence involved in transcription or stabilization of RNA. The expression control sequence may be operably linked to the sequence encoding the p53 protein. The expression control sequence may be located upstream or downstream of the sequence encoding the p53 protein. The expression control sequence may be located downstream of the sequence encoding the p53 protein and upstream of the modified polyadenylation sequence.

The expression control sequence may be a Kozak sequence, a ribosome binding sequence, or a UTR. The UTR may be a 5'UTR, a 3'UTR, or both a 5'UTR and a 3'UTR. The 5'UTR, the 3'UTR, or both the 5'UTR and the 3'UTR may be a sequence homologous or heterologous to the sequence encoding the p53 protein. The 5'UTR may be a structured UTR. That is, the 5'UTR may have a three-dimensional structure such as a stem-loop structure.

In the mRNA molecule, the 5'UTR may be a native human p53 5' UTR or a native human OX40L 5' UTR, and the 3' UTR may be a native human cytochrome P450 2E1 (CYP2E1) 3' UTR.

As used herein, the term "5'-untranslated region (5' UTR)" refers to a region of mRNA directly upstream (i.e., 5') of the translation initiation codon, which is the first codon of the mRNA transcript to be translated by the ribosome. Furthermore, the term "3'-untranslated region (3' UTR)" refers to a region of mRNA directly downstream (i.e., 3') of the stop codon that does not encode a polypeptide, which is the codon of the mRNA transcript signaling the termination of translation. The 5'UTR or 3'UTR may be found in mRNA expressed in any tissue cell. The tissue may be a tissue in which cancer is present. The cancer may be a blood cancer or a solid cancer. The tissue may be liver, muscle, brain, breast, colon, lung, pancreas, ovary, skin, or blood. The tissue cell may be, for example, a liver cell, an endothelial cell, a muscle cell, a blood cell, or an adipose tissue cell. The 5' UTR and the 3' UTR may be independently derived from an mRNA encoding a protein selected from the group consisting of: p53, OX40L, albumin, serum amyloid A, apolipoprotein A/B/E, transferrin, alpha-fetoprotein, erythropoietin, Factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen. The 5'UTR may be derived from an mRNA encoding human p53, human OX40L, or human C3 protein. Additionally, the 3'UTR may be derived from an mRNA encoding human CYP450 2E1, human OX40L, or human C3 protein. The 3'UTR may be located at the 5' to the polyadenylation sequence.

As used herein, the term "open reading frame (ORF)" or "a sequence encoding a polypeptide" refers to a continuous region of DNA that starts with an initiation codon (e.g., a methionine codon (ATG)), ends with a stop codon (e.g., TAA, TAG, or TGA), and encodes a polypeptide.

As used herein, the term "operatively linked" refers to the linkage of nucleotide sequences on a single nucleic acid fragment so that the function of one is affected by the other. For example, a promoter is operably linked to a coding sequence (e.g., an ORF) if it is capable of affecting the expression of said coding sequence (i.e., the coding sequence is under the transcriptional control of the promoter). A coding sequence may be operably linked to a regulatory sequence in a sense or antisense orientation. As used herein, unless otherwise stated, each sequence included in a nucleic acid molecule is operably linked.

The expression control sequence may be a sequence that is homologous or heterologous to the sequence encoding the p53 protein.

In the mRNA molecule, the sequence encoding the p53 protein and the modified polyadenylation sequence may be transcribed under the control of a promoter to be produced as a common transcript. The modified polyadenylation sequence may be located at the 3' end of the common transcript.

The mRNA molecule may include one or more modified nucleotides. The modified nucleotide may be a pseudouridine analogue. The pseudouridine may be 1-methylpseudouridine. The modified nucleotide may further include 5-methylcytidine. In the mRNA molecule, all U may be substituted with 1-methylpseudouridine.

The mRNA may further include a chain-terminating nucleoside. The chain-terminating nucleoside may be selected from the group consisting of 3'-deoxyadenosine (cordycepin), 3'-deoxyuridine, 3'-deoxycytidine, 3'-deoxyguanosine, 3'-deoxythymidine, 2',3'-dideoxynucleoside, 2',3'-dideoxyadenosine, 2',3'-dideoxyuridine, 2',3'-dideoxycytidine, 2',3'-dideoxyguanosine, 2',3'-dideoxythymidine, 2'-deoxynucleoside, and O-methylnucleoside.

In the mRNA molecule, the sequence encoding the p53 protein may be codon-optimized.

Additionally, the mRNA may include one or more 5'-cap structures. As used herein, the term "cap" refers to a cap structure found at the 5'-end of an mRNA molecule. The cap is generally composed of a guanosine nucleotide linked to the mRNA via a 5'-to-5' triphosphate linkage. This guanosine is methylated at the 7-position. The term "conventional 5'-cap" refers to a naturally occurring RNA 5'-cap, such as 7-methylguanosine (m7G). As used herein, the term "5'-cap" includes modified 5'-cap analogs that are similar to an RNA cap structure and, when attached, possess the ability to stabilize RNA, for example, in vivo and/or in a cell. The one or more 5'-cap structures may be selected from the group consisting of 7-methylguanosine (m7G), ARCA, inosine, Nl-methyl-guanosine, 2'-fluoro-guanosine, 7-deaza-guanosine, 8-oxo-guanosine, 2-amino-guanosine, LNA-guanosine and 2-azido-guanosine. In the mRNA, the GA at the 5' end may have a cap structure of m7(3'OMeG)(5')ppp(5')(2'OMeA). In the mRNA, all U may be substituted with N1-methyl-pseudouridine. In the mRNA, all U may be substituted with N1-methyl-pseudouridine, and a GA at the 5' terminus may have a cap structure of m7(3'OMeG)(5')ppp(5')(2'OMeA).

The 5'-cap may be Cap0, Cap1, or Cap2. In Cap0, the riboses of both the first and second cap-proximal nucleotides of the mRNA include a 2'-hydroxyl group. In Cap 1, the riboses of the first and second cap-proximal nucleotides of the mRNA include a 2'-methoxy group and a 2'-hydroxyl group, respectively. In Cap 2, the riboses of both the first and second cap-proximal nucleotides of the mRNA include a 2'-methoxy group.

Providing an RNA having a 5'-cap or a 5'-cap analog may be achieved by *in vitro* transcription of a DNA template in the presence of the 5'-cap or the 5'-cap analog. In this case, the 5'-cap or the 5'-cap analog may be incorporated into the produced RNA strand co-transcriptionally. Alternatively, for example, the RNA may be produced by in vitro transcription and may be produced post-transcriptionally using a capping enzyme, such as a capping enzyme of vaccinia virus.

A second aspect provides a method of obtaining a p53 protein, including translating the mRNA molecule.

A third aspect provides a composition for delivering an mRNA molecule or a product expressed thereby to a subject, including the mRNA molecule as an active ingredient.

The fourth aspect provides a kit for delivering an mRNA molecule or a product expressed thereby to a subject, including the mRNA molecule as an active ingredient.

In the third and fourth aspects, the composition or kit may be a pharmaceutical composition or kit for treating cancer. The cancer may be a blood cancer or a solid cancer. The above blood cancer may be leukemia, malignant lymphoma, multiple myeloma, or aplastic anemia. The solid cancer may be a brain tumor, benign astrocytoma, malignant astrocytoma, pituitary adenoma, meningioma, cerebral lymphoma, oligodendroglioma, craniopharyngioma, ependymoma, brainstem tumor, head and neck tumor, laryngeal cancer, oropharyngeal cancer, nasal cavity cancer, nasopharyngeal cancer, salivary gland cancer, hypopharyngeal cancer, thyroid cancer, oral cancer, thoracic tumor, small cell lung cancer, non-small cell lung cancer, thymic cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colorectal cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer, or skin cancer.

The composition may be administered to a subject, and the RNA (e.g., mRNA) may be translated in vivo to produce a polypeptide.

The composition may be contacted with a cell, a tissue, or a subject in an "effective amount."

The effective amount may be determined based on a target tissue, a target cell, a means of administration, physical characteristics of a polynucleotide (e.g., size and a content level of modified nucleosides), and other components of the composition.

The composition may be administered in combination with other prophylactic or therapeutic compounds. The prophylactic or therapeutic compound may be, for example, an anti-cancer agent. As used herein, the anti-cancer agent may be any known agent.

The composition may be administered intramuscularly, subcutaneously, intradermally, intranasally, or pulmonarily. The composition may be administered locally to a tissue where cancer is present. The composition may be administered locally to a tissue where cells having a high level of cancer-specific miRNA, either intracellularly or extracellularly, are present. The composition may be administered locally to a tissue where cells in which a cancer-specific miRNA is upregulated or downregulated compared to normal cells, either intracellularly or extracellularly, are present.

The composition may include a pharmaceutically acceptable carrier, diluent, or excipient. The RNA may, for example, encode a polypeptide having anticancer activity.

The composition may include one or more pharmaceutically acceptable carriers, diluents or excipients. The mRNA in the composition may be formulated or complexed with the carrier, diluent or excipient. The carrier, diluent or excipient may be known in the art.

The polynucleotide in the composition may be formulated or complexed with the carrier, diluent or excipient. The carrier, diluent or excipient may be known in the art.

The relative amounts of the active ingredient, the pharmaceutically acceptable carrier, the diluent, the excipient, and/or other additional components included in the composition may vary depending on the identity, size, and/or condition of the subject to be treated and the route of administration. For example, the composition may include 0.1% to 100%, for example, 0.5% to 50%, 1.0% to 30%, 5.0% to 80%, or 80% (w/w) or more of the active ingredient.

The composition may be formulated as nanoparticles. The nanoparticles may be those known for delivering a polynucleotide, such as mRNA, to a cell. For example, the nanoparticles may be lipid nanoparticles (LNPs). The composition may be formulated as lipid nanoparticles (LNPs) or may be bound to LNPs. The binding includes binding within or on the surface of the LNPs. For example, the composition may be formulated within a lipid polycation complex or associated with a lipid polycation complex. The lipid polycation complex is also referred to as a cationic lipid nanoparticle. The polycation may include cationic polypeptides such as MC3, Lipid 319, C12-200, 5A2-SC8, 306Oi10, Moderna Lipid 5, Acuitas A9, SM-102, ALC-0315, Arcturus Lipid 2,2(8,8) 4C CH3, Genevant CL1, polylysine, polyornithine, and/or polyarginine. In addition, the composition may be formulated in or bound to lipid nanoparticles including a non-cationic lipid, such as distearoylphosphatidylcholine (DSPC), 1,2-distearoyl-sn-glycero-3-phosphocholine (DSPC), dipalmitoylphosphatidylcholine (DPPC), a sterol such as cholesterol, or dioleoylphosphatidylethanolamine (DOPE). Furthermore, the composition may be formulated in or bound to lipid nanoparticles including a PEG-lipid, such as 1,2-dimyristoyl-rac-glycero-3-methoxypolyethylene glycol-2000 (PEG2000-DMG), 2-[(polyethylene glycol)-2000]-N,N-ditetradecylacetamide (ALC-0159), or polyethylene glycol dimethacrylate (PEG-DMA).

The lipid nanoparticle formulation may include a cationic lipid, a phospholipid, a sterol such as cholesterol, a PEG-lipid, or a combination thereof. The lipid nanoparticle formulation may include a cationic lipid, a phospholipid, a sterol such as cholesterol, and a PEG-lipid. Additionally, the lipid nanoparticle may include a PEG-modified lipid, a non-cationic lipid, a sterol, and an ionizable lipid, or a combination thereof. The lipid nanoparticles may include 0.5 to 15 mol% of the PEG-modified lipid, 5 to 25 mol% of the non-cationic lipid, 25 to 55 mol% of the sterol, and 20 to 60 mol% of the ionizable lipid. The PEG-modified lipid may be 1,2-dimyristoyl-sn-glycerol methoxypolyethylene glycol (PEG2000-DMG), the non-cationic lipid may be 1,2-distearoyl-sn-glycero-3-phosphocholine (DSPC), the sterol may be cholesterol, and the ionizable cationic lipid may be Compound 1 having the following structure:

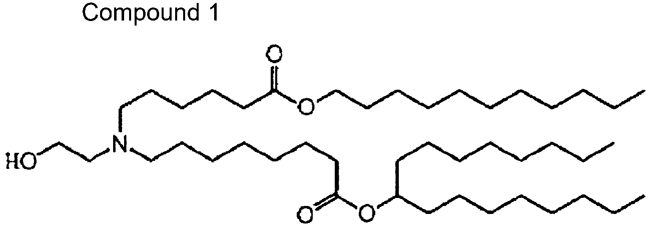

The PEG-modified lipid may be Compound 2 (ALC-0159) having the following structure, the non-cationic lipid may be 1,2 distearoyl-sn-glycerol-3-phosphocholine (DSPC), the sterol may be cholesterol, and the ionizable cationic lipid may be Compound 3 (ALC-0315) having the following structure.

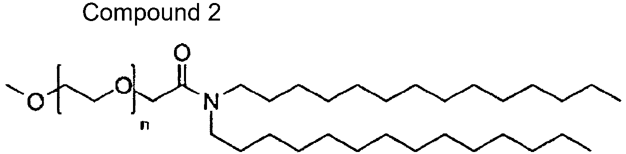

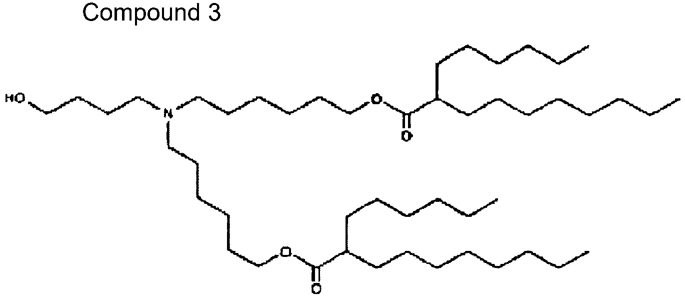

A fifth aspect provides a method of delivering an mRNA and a product expressed thereby to a subject, including introducing the mRNA into a host cell.

In the method, the host cell may be any cell derived from a subject. The subject may be a mammal, including a human. The cell may be, for example, a stem cell, a germ cell, or a somatic cell. In the method, the polynucleotide may be RNA, and the host cell may be a cancer cell, an antigen-presenting cell including a dendritic cell, a monocyte, or a macrophage. The introducing may include locally administering to a tissue where cancer is present. The introducing may include locally administering a nucleic acid molecule or an mRNA molecule to a tissue where cells having a high level of cancer-specific miRNA, either intracellularly or extracellularly, are present. The administering may include locally administering a nucleic acid molecule or an mRNA molecule to a tissue where cells in which a cancer-specific miRNA is upregulated or downregulated compared to normal cells, either intracellularly or extracellularly, are present.

The method may include administering the nucleic acid molecule or the mRNA molecule to a subject. The administrating may be parenteral or oral administration. The administrating may be intramuscular, subcutaneous, intradermal, intranasal, or intrapulmonary administration. The subject may be a mammal, including a human. The administering may include locally administering to a tissue where cancer is present. The administering may include locally administering a polynucleotide, for example, an mRNA molecule, to a tissue where cells having a high level of intracellular or extracellular cancer-specific miRNA are present. The administration may include locally administering a polynucleotide, for example, an mRNA molecule, to a tissue where cells in which a cancer-specific miRNA is upregulated or downregulated compared to normal cells, either intracellularly or extracellularly, are present.

The method may be of inhibiting cancer cell proliferation by producing the p53 protein in a cell. Additionally, the method may be for treating a disease in a subject.

A sixth aspect provides a nucleic acid molecule encoding the mRNA.

The nucleic acid molecule may be DNA. Additionally, the nucleic acid molecule may include an expression control sequence. The expression control sequence may be a transcription control sequence. The expression control sequence may be operably linked to a sequence encoding the p53 protein. The expression control sequence may be located upstream or downstream of the sequence encoding the p53 protein. The expression control sequence may be located downstream of the sequence encoding the p53 protein and upstream of the modified polyadenylation sequence.

As used herein, the term "transcription" refers to a process in which the genetic code of a DNA sequence is transcribed into RNA. The RNA may then be translated into a protein. The term "transcription" includes *in vitro* transcription. The term "*in vitro* transcription" refers to a process in which RNA, particularly mRNA, is synthesized *in vitro* in a cell-free system. Cloning vectors are applied for the production of transcripts. These cloning vectors are also generally referred to as transcription vectors. The term "vector" includes a transcription vector. The RNA is, for example, *in vitro* transcribed RNA (IVT-RNA) and may be obtained by *in vitro* transcription of a DNA template. A promoter for controlling transcription may be any promoter for any RNA polymerase. A DNA template for *in vitro* transcription may be obtained by cloning a nucleic acid, particularly a cDNA, and may be introduced into an appropriate vector for *in vitro* transcription.

The expression control sequence may be a promoter.

As used herein, the term "promoter" or "promoter region" refers to a DNA sequence upstream (5') of a coding sequence of a gene, and refers to controlling the expression of the coding sequence by providing recognition and binding sites for RNA polymerase. The promoter region may further include a recognition or binding site for additional factors involved in regulating the transcription of the gene. A promoter can control the transcription of prokaryotic or eukaryotic genes. A promoter may be "inducible," initiating transcription in response to an inducer, or "constitutive" if transcription is not controlled by an inducer. In the absence of an inducer, the transcription of a gene under the control of the inducible promoter may occur minimally or not at all. In the presence of the inducer, transcription of the gene under the control of the inducible promoter may increase.

The promoter may be derived from a homologous or heterologous source with respect to the sequence encoding the polypeptide. The promoter may be derived from a mammal, including a human. The promoter may be derived from a non-human mammal. The promoter may be derived from a plant, a microorganism, or a virus. The microorganism may be yeast or bacteria. The bacteria may be Gram-positive or Gram-negative bacteria. The bacteria may belong to the genus *Escherichia*. The promoter may be a T7, T3, or SP6 promoter. The promoter may be operable in a microorganism of the genus *Escherichia*. The microorganism may be *E. coli*.

In the nucleic acid molecule, the promoter may be a T7 promoter and the microRNA may be microRNA-34a.

The nucleic acid molecule may be a closed circular molecule or a linear molecule.

The nucleic acid molecule may further include one or more selected from the group consisting of (i) a reporter gene, (ii) a selectable marker, and (iii) an origin of replication. The nucleic acid molecule may be a vector. The vector may be a cloning vector or an expression vector.

The nucleic acid molecule may be suitable for *in vitro* transcription of mRNA after being linearized.

As used herein, the term "expression" is used in its general sense and includes the production of RNA, or RNA and protein. The expression also includes partial expression of a nucleic acid. Furthermore, the expression may be transient or stable. In the context of RNA, the term "expression" or "translation" refers to a process in the ribosomes of a cell in which a strand of messenger RNA directs the assembly of an amino acid sequence to produce a peptide or protein.

A seventh aspect provides a method of propagating a nucleic acid molecule, including: (i) providing the nucleic acid molecule; and (ii) propagating the nucleic acid molecule in a cell.

In the method, the cell may be a host cell. The term "host cell" refers to any cell into which a foreign nucleic acid may be transformed or transduced. The host cells include prokaryotic cells (e.g., *E. coli*) or eukaryotic cells (e.g., yeast cells and insect cells). The cell may be an animal, plant, or microbial cell. The animal may be a mammal. The mammals include humans, mice, rats, hamsters, pigs, and primates. The cell may be derived from a plurality of tissue cells and may include primary cells and cell lines. The microorganism may be yeast or bacteria. The bacteria may be Gram-positive or Gram-negative bacteria. The bacteria may belong to the genus *Escherichia*. The cell may be *E. coli*.

In the method, the proliferating may include transforming a cell with the nucleic acid molecule and culturing the transformed cell.

The method may include isolating the nucleic acid molecule from the cell after propagation.

An eighth aspect provides a method of obtaining RNA, including: (i) propagating a nucleic acid molecule according to the method of the seventh aspect; and (ii) *in vitro* transcribing the nucleic acid molecule into RNA as a template.

The term "*in vitro* transcription" or "RNA *in vitro* transcription" refers to a process in which RNA, including mRNA, is synthesized in a cell-free system, i.e., *in vitro*. Cloning vector DNA, including plasmid DNA vectors, may be applied as a template for the production of RNA transcripts. These cloning vectors are also generally referred to as transcription vectors. The RNA may be obtained by DNA-dependent *in vitro* transcription of an appropriate DNA template. The DNA template may be a linearized plasmid DNA template. A promoter for controlling RNA *in vitro* transcription may be any promoter for a DNA-dependent RNA polymerase. The DNA-dependent RNA polymerase may be T7 RNA polymerase, T3 RNA polymerase, SP6 RNA polymerase, or a combination thereof. A DNA template for RNA *in vitro* transcription may be obtained by cloning a nucleic acid and introducing it into a vector for RNA *in vitro* transcription. The DNA may include a cDNA corresponding to each RNA transcribed *in vitro*. The vector for RNA *in vitro* transcription may be a circular plasmid DNA. The cDNA may be obtained by reverse transcription of mRNA or by chemical synthesis.

A ninth aspect provides a method of obtaining a p53 protein, including: (i) obtaining an mRNA encoding the p53 protein according to the method of the eighth aspect; and (ii) translating the mRNA into a p53 protein.

The method may include, prior to (i), cleaving the nucleic acid molecule.

A tenth aspect provides an RNA molecule obtained by transcribing the nucleic acid molecule of the sixth aspect. The RNA molecule may be an mRNA molecule.

### Advantageous Effects

The mRNA molecule according to an aspect is relatively stable and has high translation efficiency, and thus may be used to efficiently translate mRNA.

The nucleic acid molecule according to an aspect may be used to prepare RNA with increased stability.

According to a method of propagating a nucleic acid molecule according to an aspect, a nucleic acid molecule may be efficiently propagated.

According to a method of obtaining an RNA according to an aspect, RNA may be obtained efficiently.

According to a method of obtaining p53 protein according to an aspect, p53 protein may be obtained efficiently.

The mRNA molecule according to an aspect has increased stability and may be used for expression at an increased level.

A composition or kit for delivering an mRNA or a product expressed thereby to a subject according to an aspect may be used to treat cancer.

According to the method of delivering an mRNA or a product expressed thereby to a subject according to an aspect, the mRNA or the product expressed thereby may be efficiently delivered to the subject.

### Description of Drawings

FIG. 1 is a view showing a vector obtained by the same processes as in Examples 1 and 2.
FIG. 2 is a view showing a vector including a T7 promoter, a 5' UTR, and a target protein ORF between HindIII and XhoI restriction enzyme sites in the template vector of FIG. 1.
FIG. 3 is a view showing the p53 gene expression vector, p53-polyA-miR34a, including a 5' UTR, a p53 gene, a 3' UTR, and a modified polyadenylation sequence including miR34a.
FIG. 4 is a view showing the results of confirming, through Western blot analysis, the effect of miRNA-34a on the expression of mRNA introduced into cells.
FIG. 5A is a view showing the degree of growth inhibition of a normal cell line, BEAS-2B, by p53-polyA-miR34a mRNA.
FIG. 5B is a view showing the degree of growth inhibition of a lung cancer cell line, H1373, by p53-polyA-miR34a mRNA.
FIG. 5C is a view showing the degree of growth inhibition of a lung cancer cell line, H1299, by p53-polyA-miR34a mRNA.

### Best Mode

Hereinafter, the present disclosure will be described in more detail with reference to examples. However, these examples are for illustrative purposes only, and the scope of the present disclosure is not limited to these examples.

### Example 1: Cloning of 3'UTR Sequence

In this example, 3'UTR was cloned to increase the expression level and sequence stability of the gene encoding mRNA. The 3' UTR sequence of human cytochrome P450 2E1 was used as the 3' UTR. The 3' UTR was prepared to include XhoI and NheI restriction enzyme sequences at the 5'-end and 3'-end, respectively, to be used for recombinant nucleic acid construction. The nucleic acid may be an open reading frame (ORF). The 3'UTR sequence may be linked to the 3'-end of the open reading frame through the XhoI restriction enzyme site at the 5'-end.

A recombinant 3' UTR sequence including the XhoI restriction enzyme sequence, the 3' UTR sequence of human cytochrome P450 2E1, and the NheI restriction enzyme sequence was synthesized by an in vitro nucleic acid synthesis method. The obtained recombinant 3' UTR sequence is DNA. After treating and cleaving both the recombinant 3' UTR sequence and a template vector with XhoI and NheI restriction enzymes, respectively, both sequences were linked using DNA ligase. A pUC57-KanR vector (selfproduced) was used as the template vector. The pUC57-KanR vector is a pUC57 vector including Kanamycin as an antibiotic resistance gene. The antibiotic resistance gene is a selectable marker. As a result, a template vector including the recombinant 3' UTR sequence was constructed. Table 1 shows the sequence encoding the 3'UTR sequence of human cytochrome P450 2E1.

**[Table 1]**

| SEQ ID NO: | 3'UTR Sequence |
|---|---|
| 1 | GTGTGTGGAGGACACCCTGAACCCCCCGCTTTCAAACAAGTTTTCA AATTGTTTGAGGTCAGGATTTCTCAAACTGATTCCTTTCTTTGCATAT GAGTATTTGAAAATAAATATTTTCCCAGAATATAAATAAATCATCACA TGATTATTTTAACTAT |

In Table 1, the sequence of SEQ ID NO: 1 is the wild-type 3' UTR sequence of the human cytochrome P450 2E1 gene.

### Example 2: Cloning of Consecutive Adenosine Sequence Including miRNA Binding Site

In this example, a recombinant template vector was constructed in which a modified polyadenylation sequence was linked to the 3'-end of the 3' UTR sequence in the recombinant 3' UTR-containing template vector prepared in Example 1. The modified polyadenylation sequence is intended to increase the stability and expression level of a gene encoding mRNA.

The modified polyadenylation sequence includes a nucleotide sequence complementary to miRNA-34a in the middle region of an unmodified 100 nt polyadenylation sequence. Specifically, the modified polyadenylation sequence is one in which the sequences from positions 1 to 22 of the nucleotide sequence complementary to miRNA-34a are introduced between positions 20 and 21 of the unmodified polyadenylation sequence.

In addition, the modified polyadenylation sequence was designed to include NheI and EcoRI restriction enzyme sequences at the 5'-end and the 3'-end, respectively, to be used for recombination. A recombinant modified polyadenylation sequence including the Nhe I restriction enzyme sequence, the modified polyadenylation sequence, and the EcoRI restriction enzyme sequence was synthesized by an *in vitro* nucleic acid synthesis method. The obtained recombinant sequence is DNA.

The obtained recombinant modified polyadenylation sequence and the recombinant 3' UTR-containing template vector prepared in Example 1 were treated and cleaved with Nhe I and Eco RI restriction enzymes, respectively, and both sequences were linked using DNA ligase to construct a template vector containing a 3' UTR-modified polyadenylation sequence. Table 2 shows the nucleotide sequence of the modified polyadenylation sequence.

**[Table 2]**

| SEQ ID NO: | Poly(A) Tail Sequence |
|---|---|
| 2 | AAAAAAAAAAAAAAAAAAAAAACAACCAGCTAAGACACTGCCAAAAAA AAAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAA |

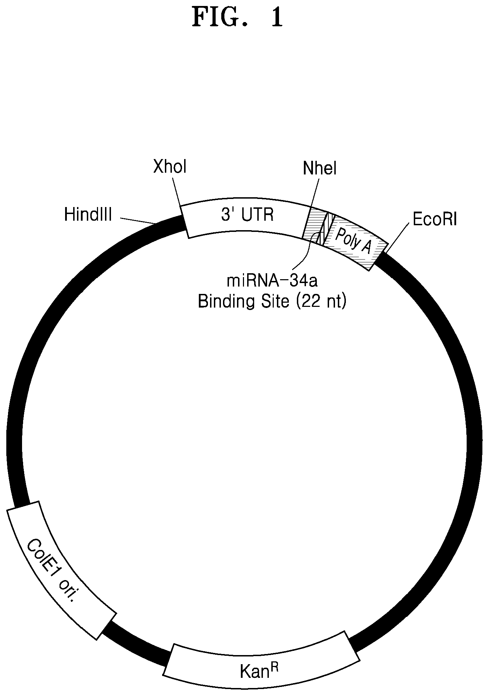

The underlined nucleotides in the sequences listed in Table 2 are sequences complementary to miRNA-34a, i.e., miRNA-34a binding site. FIG. 1 is a view showing a vector obtained by the same processes as in Examples 1 and 2. As shown in FIG. 1, the vector includes a 3' UTR linked to the 5' end of a modified polyadenylation sequence including the miRNA-34a binding sequence in the region of positions 21 to 42 of its nucleotide sequence. The vector includes HindIII and XhoI restriction enzyme recognition sites upstream of the 3' UTR, and thus may be used to introduce a target protein ORF linked to the 3' end of a 5' UTR. That is, the vector may be a platform vector used for cloning an ORF. The 5' UTR-ORF is linked to a T7 promoter upstream thereof. The vector also includes an antibiotic resistance marker (KanR) and an *E. coli* origin of replication (ColE1 ori).

The nucleotide sequence of the template vector as shown in FIG. 1 is shown in Table 3.

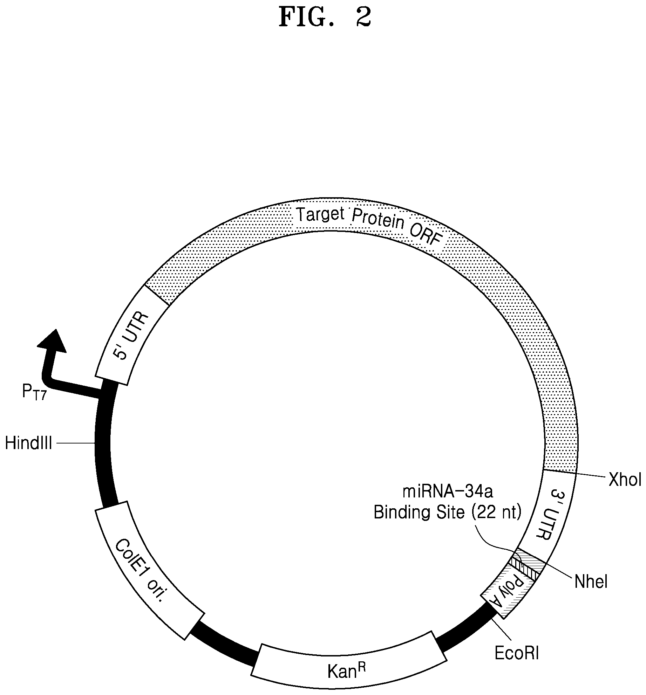

FIG. 2 is a view showing a vector including a T7 promoter, a 5' UTR, and a target protein ORF between HindIII and XhoI restriction enzyme sites in the template vector of FIG. 1.

**[Table 3]**

| SEQ ID NO: | Nucleotide Sequence |
|---|---|
| 3 | AAGCTTCTCGAGgtgtgtggaggacaccctgaaccccccgctttcaaacaagttttcaaattgttt gaggtcaggatttctcaaactgattcctttctttgcatatgagtatttgaaaataaatattttcccagaatata aataaatcatcacatgattattttaactatgctagcaAAAAAAAAAAAAAAAAAAAacaacc agctaagacactgccaAAAAAAAAAAAAAAAAAAAAAAAAAAAAAAAAAA AAAAAAAAAAAAAAAAAAAAAAAAAAAAAAAAAAAAAAAAAAAAAA AAAAAAAAAAAAAAAGAAGAGCGAATTCTCAAGACGATAGTTACCGGAT AAGGCGCAGCGGTCGGGCTGAACGGGGGGTTCGTGCACACAGCCCA GCTTGGAGCGAACGACCTACACCGAACTGAGATACCTACAGCGTGAG CTATGAGAAAGCGCCACGCTTCCCGAAGGGAGAAAGGCGGACAGGT ATCCGGTAAGCGGCAGGGTCGGAACAGGAGAGCGCACGAGGGAGCT TCCAGGGGGAAACGCCTGGTATCTTTATAGTCCTGTCGGGTTTCGCC ACCTCTGACTTGAGCGTCGATTTTTGTGATGCTCGTCAGGGGGGCGG AGCCTATGGAAAAACGCCAGCAACGCGGCCTTTTTACGGTTCCTGGC CTTTTGCTGGCCTTTTGCGAGCCTTTTGCTCACATGTTCTTTCCTGCG TTATCCCCTGATTCTGTGGATAACCGTATTACCGCCTTTGAGTGAGCT GATACCGCTCGCCGCAGCCGAACGACCGAGCGCAGCGAGTCAGTGA GCGAGGAAGCGGCCCAATACGCAAACCGCCTCTCCCCGCGCGTTGG CCGATTCATTAATGCAGCTGGCACGACAGGTTTCCCGACTGGAAAGC GGGCAGTGAGCGCAACGCAATTAATGTGAGTTAGCTCACTCATTAGG CACCCCAGGCTTTACACTTTATGCTTCCGGCTCGTATGTTGTGTGGAA TTGTGAGCGGATAACAATTTCACACAGGAAACAGCTATGACCATGATT ACGCC |
| 4 | gtgtgtggaggacaccctgaaccccccgctttcaaacaagttttcaaattgtttgaggtcaggatttctca aactgattcctttctttgcatatgagtatttgaaaataaatattttcccagaatataaataaatcatcacatga ttattttaactatAAAAAAAAAAAAAAAAAAAaacaaccagctaagacactgccaGCCAC CACTTCAAGAACTCTGTAGCACCGCCTACATACCTCGCTCTGCTAATC CTGTTACCAGTGGCTGCTGCCAGTGGCGATAAGTCGTGTCTTACCGG GTTGGACTCAAGACGATAGTTACCGGATAAGGCGCAGCGGTCGGGC TGAACGGGGGGTTCGTGCACACAGCCCAGCTTGGAGCGAACGACCT ACACCGAACTGAGATACCTACAGCGTGAGCTATGAGAAAGCGCCACG CTTCCCGAAGGGAGAAAGGCGGACAGGTATCCGGTAAGCGGCAGGG TCGGAACAGGAGAGCGCACGAGGGAGCTTCCAGGGGGAAACGCCTG GTATCTTTATAGTCCTGTCGGGTTTCGCCACCTCTGACTTGAGCGTCG ATTTTTGTGATGCTCGTCAGGGGGGCGGAGCCTATGGAAAAACGCCA GCAACGCGGCCTTTTTACGGTTCCTGGCCTTTTGCTGGCCTTTTGCG AGCCTTTTGCTCACATGTTCTTTCCTGCGTTATCCCCTGATTCTGTGG ATAACCGTATTACCGCCTTTGAGTGAGCTGATACCGCTCGCCGCAGC CGAACGACCGAGCGCAGCGAGTCAGTGAGCGAGGAAGCGGCCCAAT ACGCAAACCGCCTCTCCCCGCGCGTTGGCCGATTCATTAATGCAGCT GGCACGACAGGTTTCCCGACTGGAAAGCGGGCAGTGAGCGCAACGC AATTAATGTGAGTTAGCTCACTCATTAGGCACCCCAGGCTTTACACTT TATGCTTCCGGCTCGTATGTTGTGTGGAATTGTGAGCGGATAACAATT TCACACAGGAAACAGCTATGACCATGATTACGCC |

In the sequence of the template vector of SEQ ID NO: 3 described in Table 3, the nucleotides indicated by underlined uppercase letters are the Hind III restriction enzyme recognition sequence. In the sequence of the template vector of SEQ ID NO: 3 described in Table 3, the nucleotides indicated by bold uppercase letters are the Xho I restriction enzyme recognition sequence. In the sequence of the template vector of SEQ ID NO: 3 described in Table 3, the nucleotides indicated by lowercase letters are the wild-type 3' UTR sequence of human cytochrome P450 2E1 (SEQ ID NO: 1).

In the sequence of the template vector of SEQ ID NO: 3 described in Table 3, the nucleotides indicated by bold lowercase letters are the Nhe I restriction enzyme recognition sequence.

In the sequence of the template vector of SEQ ID NO: 3 described in Table 3, the nucleotides indicated by underlined lowercase letters are the miRNA-34a binding sequence complementary to miRNA-34a.

In the sequence of the template vector of SEQ ID NO: 3 described in Table 3, the nucleotides indicated by italicized uppercase letters are the Eco RI restriction enzyme recognition sequence.

SEQ ID NO: 4 in Table 3 is a sequence of SEQ ID NO: 3 excluding the restriction enzyme recognition sites.

### Example 3: Construction of a p53 mRNA Transcription Vector

In this example, a template vector to be used for p53 mRNA transcription was constructed using the vector platform as shown in FIG. 1, which was prepared in Example 2.

### Example 3-1: Cloning of 5' UTR Sequence and Wild-type p53 Gene Sequence

A recombinant gene including a 5' restriction enzyme recognition site, a T7 promoter sequence, a 5' UTR, an open reading frame (ORF), and a 3' restriction enzyme recognition site was synthesized by an *in vitro* nucleic acid synthesis method. The 5' restriction enzyme recognition site and the 3' restriction enzyme recognition site are HindIII and XhoI restriction enzyme sequences, respectively. The 5' UTR is the sequence indicated in bold in Table 4 below. The open reading frame (ORF) is a nucleotide sequence encoding human wild-type p53. As a result, a recombinant sequence was obtained in which the HindIII recognition site, the T7 promoter sequence, the 5' UTR, the p53 ORF, and the XhoI recognition site were linked.

The obtained recombinant sequence and the template vector of FIG. 1 prepared in Example 2 were treated and cleaved with the restriction enzymes Hind III and Xho I, respectively, and both sequences were linked using DNA ligase. As a result, a transcription vector including the 5' UTR, the wild-type p53 ORF, the cytochrome P450 2E1 3' UTR, and the modified polyadenylation sequence was constructed (hereinafter referred to as "p53-polyA-miR34a"). As a control, a vector in which the modified polyadenylation sequence in the p53-polyA-miR34a was substituted with an unmodified poly(A) including 100 As was used (hereinafter referred to as "p53-polyA").

Table 4 shows the nucleotides of the recombinant sequence in which the HindIII recognition site, the T7 promoter sequence, the 5' UTR, the p53 ORF, and the XhoI recognition site are linked. The HindIII and XhoI recognition sites were attached to both ends of the obtained linked recombinant sequence.

**[Table 4]**

| SEQ ID NO: | 5' UTR and Wild-Type p53 Gene Sequence |
|---|---|
| 5 | TAATACGACTCACTATA |
| 6 | AGTGCTCCCCATCCAACTAAACTGTCCCTCTGTCCGAACTAAACTGCA CTGTCCCAGCACC |
| 7 | TCCGCGAATTGAACGAGGCCCTGGAGTTGAAAGACGCTCAAGCCGGAA AGGAACCAGGTGGGTCTAGGGCCCACTCAAGCCATCTGAAGAGTAAGA AAGGCCAGTCCACTTCCAGGCACAAAAAGTTAATGTTCAAGACGGAGGG TCCTGACTCTGAC |

In the sequence described in Table 4, the underlined nucleotides of SEQ ID NO: 5 refer to the T7 promoter region involved in the initiation of mRNA transcription. In addition, the nucleotides indicated in bold in SEQ ID NO: 6 are the 5' UTR sequence. Furthermore, SEQ ID NO: 7 is a nucleotide sequence downstream of the 5' UTR, which encodes the wild-type p53 protein.

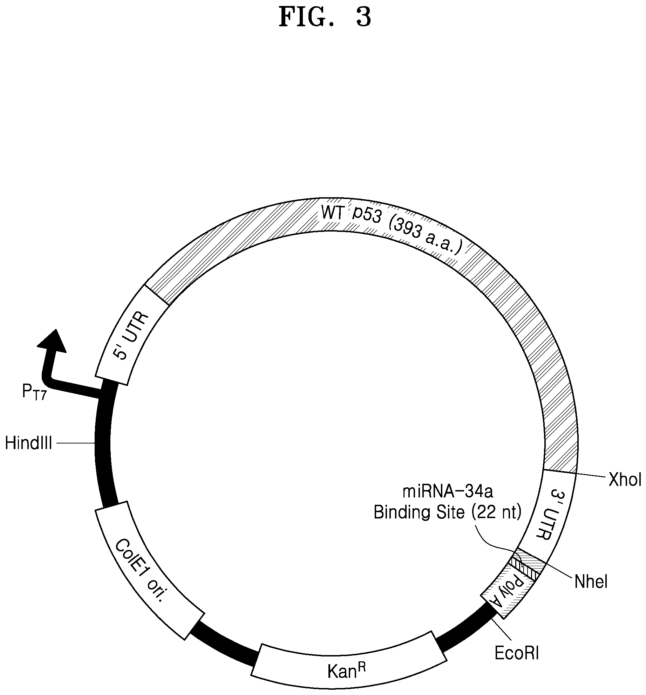

FIG. 3 is a view showing a p53 gene expression vector, p53-polyA-miR34a, including a 5' UTR, a p53 gene, a 3' UTR, and a modified polyadenylation sequence including miR34a. Table 5 shows the nucleotide sequence of each element among products obtained by *in vitro* transcription of the obtained transcription vector. Table 6 shows the amino acid sequence encoded by the p53 gene included in the obtained transcription vector.

**[Table 5]**

| SEQ ID NO: | Region | mRNA Sequence |
|---|---|---|
| 8 | 5'UTR | AGUGCUCCCCAUCCAACUAAACUGUCCCUCUGUCC GAACUAAACUGCACUGUCCCAGCACC |
| 9 | Wild-type p53 Protein | UCCGCGAAUUGAACGAGGCCCUGGAGUUGAAAGAC GCUCAAGCCGGAAAGGAACCAGGUGGGUCUAGGGC CCACUCAAGCCAUCUGAAGAGUAAGAAAGGCCAGU CCACUUCCAGGCACAAAAAGUUAAUGUUCAAGACGG AGGGUCCUGACUCUGAC CUCGAG |
| 10 | 3'UTR | GUGUGUGGAGGACACCCUGAACCCCCCGCUUUCAA ACAAGUUUUCAAAUUGUUUGAGGUCAGGAUUUCUC AAACUGAUUCCUUUCUUUGCAUAUGAGUAUUUGAAA AUAAAUAUUUUCCCAGAAUAUAAAUAAAUCAUCACA UGAUUAUUUUAACUAUGCUAGC |
| 11 | Poly(A) Tail | AAAAAAAAAAAAAAAAAAAAAACAACCAGCUAAGACAC UGCCAAAAAAAAAAAAAAAAAAAAAAAAAAAAAAAAAAAA AAAAAAAAAAAAAAAAAAAAAAAAAAAAAAAAAAAAAAAA AAAAAAAAAAAAAAAAAAAAAAAAAAAAAAAAGAAGAGC GAAUU |
| 12 | Wild-type p53 Protein | UCCGCGAAUUGAACGAGGCCCUGGAGUUGAAAGAC GCUCAAGCCGGAAAGGAACCAGGUGGGUCUAGGGC CCACUCAAGCCAUCUGAAGAGUAAGAAAGGCCAGU CCACUUCCAGGCACAAAAAGUUAAUGUUCAAGACGG AGGGUCCUGACUCUGAC |
| 13 | 3'UTR | GUGUGUGGAGGACACCCUGAACCCCCCGCUUUCAA ACAAGUUUUCAAAUUGUUUGAGGUCAGGAUUUCUC AAACUGAUUCCUUUCUUUGCAUAUGAGUAUUUGAAA AUAAAUAUUUUCCCAGAAUAUAAAUAAAUCAUCACA UGAUUAUUUUAACUAU |
| 14 | Poly(A) Tail | AAAAAAAAAAAAAAAAAAAAACAACCAGCUAAGACAC UGCCAAAAAAAAAAAAAAAAAAAAAAAAAAAAAAAAAA |
| | | AAAAAAAAAAAAAAAAAAAAAAAAAAAAAAAAAAAAAAAA AAAAAAAAAAAAAAAAAAAAAAAAAAAAAA |

In the wild-type p53 protein region of SEQ ID NO: 9 described in Table 5, the underlined nucleotides are the Xho I restriction enzyme recognition sequence. In the 3' UTR region of SEQ ID NO: 10 described in Table 5, the underlined nucleotides are the Nhe I restriction enzyme recognition sequence.

In the poly(A) tail region of SEQ ID NO: 11 described in Table 5, the underlined nucleotides are the Sap I restriction enzyme recognition sequence.

In the poly(A) tail region of SEQ ID NO: 11 described in Table 5, the bold nucleotides are a part of the Eco RI restriction enzyme recognition sequence.

SEQ ID NOs: 12 to 14 described in Table 5 are sequences excluding the restriction enzyme recognition parts from SEQ ID NOs: 9 to 11, respectively.

**[Table 6]**

| SEQ ID NO: | Wild-type p53 Protein Amino Acid Sequence |
|---|---|
| 15 | |

In Table 6, SEQ ID NO: 15 is the amino acid sequence obtained by translation of SEQ ID NO: 12.

### Example 3-2: Confirmation of p53 mRNA Expression in H1299 Cells

An *in vitro* transcription reaction was performed by incubating a mixture containing the p53-polyA-miR34a vector and the p53-polyA vector prepared in Example 3-1, respectively, to obtain p53-polyA-miR34a mRNA and p53-polyA mRNA. The p53-polyA-miR34a mRNA includes the 5' UTR, the p53 ORF, the 3' UTR, and the polyA-miR34a of SEQ ID NOs: 8, 9, 10, and 11.

The obtained p53-polyA-miR34a mRNA or p53-polyA mRNA, and miRNA-34a were delivered to H1299 cells, and their expression was confirmed. A synthetic miRNA-34a was used. The H1299 cell line is a human non-small cell lung carcinoma (NSCLC) cell line derived from a lymph node. H1299 cells do not express the p53 protein due to a homozygous partial deletion of the p53 gene (hereinafter referred to as "p53-null lung cancer cells").

Specifically, H1299 cells were seeded in each well of a 6-well plate at a density of 3x105 cells/2 mL medium/well and incubated at 37 °C for 24 hours. RPMI 1640 (Gibco, Cat No. 22400-089) was used as the culture medium. Next, the p53-polyA-miR34a mRNA or p53-polyA mRNA and Lipofectamine^{™} MessengerMAX^{™} Transfection Reagent (Thermo Scientific^{™}, Cat No. LMRNA001) were mixed at a ratio of 1 µL per 1 µg of mRNA and incubated for 20 minutes. 1 µg of the obtained reaction product and miRNA-34a were added to each well of the 6-well plate. The miRNA-34a was added at concentrations of 0 nM, 10 nM, and 25 nM, respectively. The reaction mixture was cultured at 37 °C for 24 hours. After completion of the culture, 100 µL/well of a lysis buffer, M-PER^{™} (Mammalian Protein Extraction Reagent) (Thermo Scientific^{™}, Cat No. 78501), was added to each well to lyse the cells and extract proteins. Subsequently, protein quantification was performed using the Pierce^{™} BCA Protein Assay Kit (Thermo Scientific^{™}, Cat No. 23225). The obtained samples were diluted with lysis buffer and a 5x reducing dye (10% SDS, 0.5% bromophenol blue, 50% glycerol, 0.5% 2-mercaptoethanol, and 250 mM Tris, pH 6.8) to equalize the protein concentrations. Western blot analysis was performed on the obtained diluted samples using an anti-p53 monoclonal antibody (CST, Cat No. #2527) and an anti-β-actin antibody (Abcam, Cat No. ab8227).

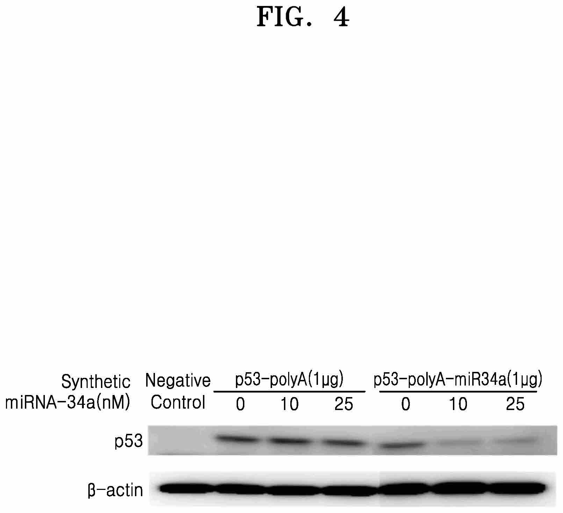

FIG. 4 is a view showing the results of confirming the effect of miRNA-34a on the expression of mRNA introduced into cells through Western blot analysis. As shown in FIG. 4, the expression of p53 was significantly reduced in p53-polyA-miR34a mRNA compared to p53-polyA mRNA. This indicates that the expression of p53 is decreased by the modified poly(A) including the miR34a binding site.

Specifically, since the p53-polyA mRNA does not have a miRNA-34a binding site at the poly(A) position, there was no difference in p53 expression even when the concentration of the synthetic miRNA-34a increased. On the other hand, the p53-polyA-miR34a mRNA possesses a site where miRNA-34a may bind at the poly(A) position, and thus the expression of p53 decreased in proportion to the concentration of the added synthetic miRNA-34a. This indicates that miRNA-34a normally binds to the miRNA-34a binding site inserted at the poly(A) position to regulate p53 expression. However, the claimed invention is not limited by a specific mechanism.

### Example 3-3: Confirmation of Cell Growth Inhibition in BEAS-2B, H1373, and H1299 Cells

An *in vitro* transcription reaction was performed by incubating a mixture containing the p53-polyA-miR34a vector prepared in Example 3-1 to obtain p53-polyA-miR34a mRNA. The p53-polyA-miR34a mRNA includes the 5' UTR, the p53 ORF, the 3' UTR, and the polyA-miR34a of SEQ ID NOs: 8, 9, 10, and 11.

The obtained p53-polyA-miR34a mRNA was delivered to normal cells and cancer cells, and its cell growth inhibitory activity was confirmed. BEAS-2B, a human bronchial epithelial cell line, was used as the normal cell line, and H1373 and H1299 cells were used as the cancer cell lines. The H1373 cell line is a lung adenocarcinoma cell line in which the p53 gene is mutated and its expression is not detected by Western blot.

Specifically, BEAS-2B, H1373, and H1299 cells were seeded in each well of a 96-well plate at densities of 3x103 cells/200 µL medium/well, 3x103 cells/200 µL medium/well, and 5x103 cells/200 µL medium/well, respectively, and incubated at 37 °C for 24 hours. For the culture medium, BEBM (Lonza, Cat No. CC-3171) was used for BEAS-2B cells, and RPMI 1640 (Gibco, Cat No. 22400-089) was used for H1373 and H1299 cells. Next, the p53-polyA-miR34a mRNA and Lipofectamine^{™} MessengerMAX^{™} Transfection Reagent (Thermo Scientific^{™}, Cat No. LMRNA001) were mixed at a ratio of 1 µL per 1 µg of the p53-polyA-miR34a mRNA and incubated for 20 minutes. 0.2 µL of the resulting reaction product was added to each well of the 96-well plate. The p53-polyA-miR34a mRNA was added at eight concentrations, starting from 4 µg/mL with 4-fold serial dilutions. After culturing the cells in the medium of each well of the resulting plate at 37 °C for 3 days, cell viability was measured using the WST-8 cell viability assay kit (BIOMAX, Cat No. QM5000).

Upon completion of the culture, the WST-8 reagent was mixed with the cell culture medium at a ratio of 1:10 and allowed to react for 2 hours. Subsequently, the absorbance at a wavelength of 450 nm was measured, and the GI₅₀ value was derived using Graphpad PrismTM software. The GI₅₀ represents the half maximal growth inhibition concentration.

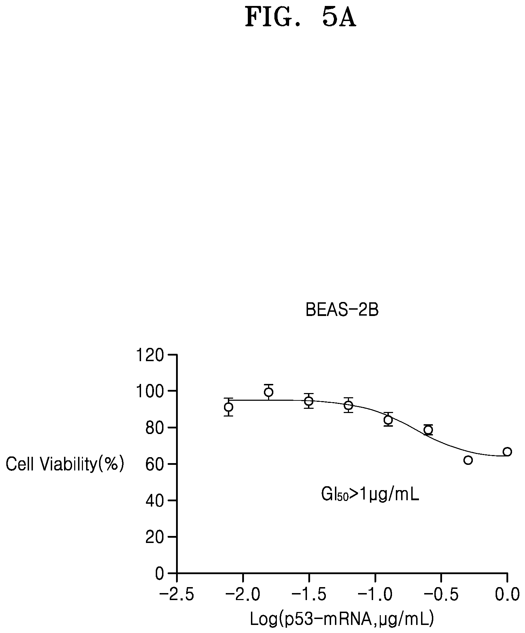

FIG. 5A is a view showing the degree of growth inhibition of the normal cell line, BEAS-2B, by p53-polyA-miR34a mRNA.

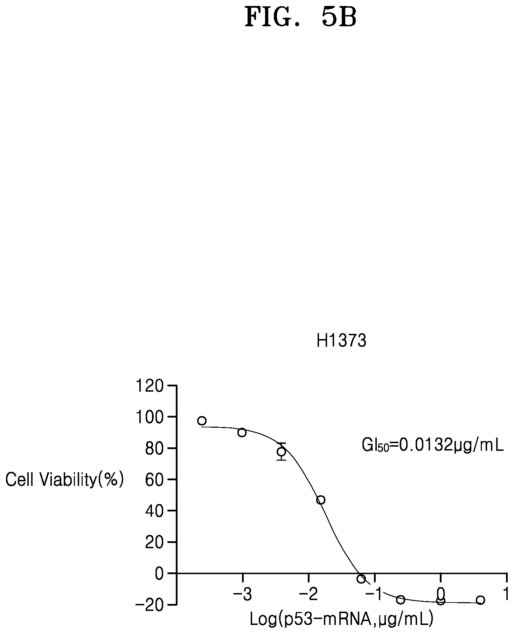

FIG. 5B is a view showing the degree of growth inhibition of the lung cancer cell line, H1373, by p53-polyA-miR34a mRNA.

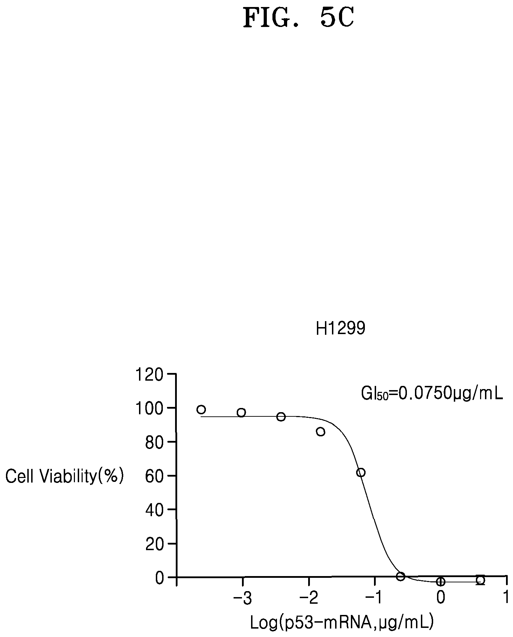

FIG. 5C is a view showing the degree of growth inhibition of the lung cancer cell line, H1299, by p53-polyA-miR34a mRNA.

As shown in FIGS. 5A, 5B, and 5C, the mRNA into which the miRNA binding site was introduced exhibited almost no growth inhibition in normal cells, while specifically inhibiting cell growth in cancer cells.

## Claims

1. An mRNA molecule comprising a p53 protein coding sequence and a modified polyadenylation sequence,
wherein the modified polyadenylation sequence comprises: (a) a first sequence comprising one or more microRNA binding sequences;
(b) a second sequence of 20 or fewer consecutive A nucleotides, located at a 5' end of the first sequence; and
(c) an mRNA molecule comprising a third sequence of 80 or more consecutive A nucleotides, located at a 3' end of the first sequence.

2. The mRNA molecule of claim 1, wherein the microRNA binding sequence is tumor-specific.

3. The mRNA molecule of claim 2, wherein the microRNA is selected from the group consisting of miRNA-34a, miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p.

4. The mRNA molecule of claim 1, further comprising an expression control sequence operably linked to the mRNA molecule.

5. The mRNA molecule of claim 4, wherein the expression control sequence is a Kozak sequence, a 5' UTR, a 3' UTR, or both 5' UTR and 3' UTR sequences.

6. The mRNA molecule of claim 5, wherein the 5' UTR is derived from an mRNA encoding a protein selected from the group consisting of p53, OX40L, albumin, serum amyloid A, apolipoprotein A/B/E, transferrin, alpha-fetoprotein, erythropoietin, Factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen.

7. The mRNA molecule of claim 5, wherein the 3' UTR is derived from an mRNA encoding a protein selected from the group consisting of p53, OX40L, albumin, serum amyloid A, apolipoprotein A/B/E, transferrin, alpha-fetoprotein, erythropoietin, Factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen.

8. The mRNA molecule of claim 1, wherein the modified polyadenylation sequence comprises 90 to 1125 nucleotides.

9. The mRNA molecule of claim 1, comprising one or more modified nucleotides.

10. A composition for delivering, to a subject, mRNA or a product expressed thereby, comprising the mRNA of any one of claims 1 to 9 as an active ingredient.

11. A method of delivering mRNA or a product expressed thereby to a host cell, comprising introducing the mRNA of any one of claims 1 to 9 into the host cell.

12. A DNA molecule encoding any one of the mRNA molecules of claims 1 to 9.

13. The DNA molecule of claim 12, further comprising an expression control sequence operably linked to the nucleic acid sequence, wherein the expression control sequence comprises a T7, T3, or SP6 promoter.

14. The DNA molecule of claim 12, wherein the DNA molecule is a nucleic acid construct or vector.
